# EUROPÄISCHE PATENTSCHRIFT

(11) **EP 1 945 602 B1**
(45) Veröffentlichungstag und Bekanntmachung des Hinweises auf die Patenterteilung: **01.08.2012**
(21) Anmeldenummer: 06807523.3
(22) Anmeldetag: 25.10.2006
(51) Int. Cl.: C07C 67/02, C07C 69/54, C07D 207/26, C08F 265/04, C08L 51/00, C07D 207/27, C08F 20/00

(54) **HYDROGEN KATALYSIERTES VERFAHREN ZUR HERSTELLUNG VON (METH)ACRYLATEN VON N-HYDROXYALKYLIERTEN LACTAMEN**
HYDROGEN-CATALYSED PROCESS FOR PREPARING (METH)ACRYLATES OF N-HYDROXYALKYLATED LACTAMS
PROCEDE CATALYSE PAR L HYDROGENE DE FABRICATION DE (METH)ACRYLATES A PARTIR DE LACTAMES N-HYDROXYALKYLES

(30) Priorität: 03.11.2005 DE 102005052931
(43) Veröffentlichungstag der Anmeldung: 23.07.2008
(73) Patentinhaber: BASF SE, 67056 Ludwigshafen (DE)
(72) Erfinder: HÖFER, Frank, 67098 Bad Dürkheim (DE); BERGMANN, Hermann, 68159 Mannheim (DE)
(86) Internationale Anmeldenummer: PCT/EP2006/067740
(87) Internationale Veröffentlichungsnummer: WO 2007/051738

(56) Entgegenhaltungen:
- WO-A-03/006568
- GB-A- 930 668
- US-A- 3 067 163

## Beschreibung

Die vorliegende Erfindung betrifft ein Verfahren zur katalytischen Herstellung von (Meth)acrylaten von N-hydroxyalkylierten Lactamen und deren Verwendung.

Die Herstellung von (Meth)acrylsäureestern erfolgt zumeist durch katalytische Veresterung von (Meth)acrylsäure oder Umesterung von anderen (Meth)acrylsäureestern mit Alkoholen in Gegenwart starker Säuren oder starker Basen. Säure- oder baseempfindliche (Meth)acrylsäureester lassen sich daher durch eine Ver- oder Umesterung in der Regel nicht gezielt herstellen.

WO 03/6568 beschreibt die saure Veresterung von Acrylsäure mit Hydroxyethylpyrrolidon mit lediglich 71 % Ausbeute.

Aus NL 6506333 und GB 930668 ist die Umesterung von N-Hydroxyalkyl lactamen mit (Meth)Acrylsäureestern in Gegenwart von Alkalimetall oder Ammonium alkoholaten sowie Titantetraalkoholaten bekannt.

Nachteilig an diesen Verfahren ist, daß die Metallverbindungen feuchtigkeitkeitsempfindlich sind und daher leicht desaktivieren. Zudem beinflussen im Produkt verbleibende Spuren der Katalysatoren eine eventuell nachfolgende Polymerisation, und müssen daher aufwendig aus dem Produkt entfernt werden. Eine solche Entfernung wird zumeist mittels einer wäßrigen Wäsche durchgeführt, so daß das Produkt anschließend getrocknet werden muß.

Aus GB 930668 ist zusätzlich die Herstellung von Lactam(meth)acrylaten durch Reaktion von N-Hydroxyalkyl lactamen mit (Meth)Acrylsäurechlorid bekannt.

Die Verwendung von (Meth)Acrylsäurechlorid bei den beschriebenen Umsetzungen führt zur Salzbildung und wegen dessen hoher Reaktivität zu unselektiven Reaktionen, wie beispielsweise Michael-Additionen.

Aufgabe der vorliegenden Erfindung war es, ein weiteres Verfahren zur Verfügung zu stellen, mit dem (Meth)acrylate von N-hydroxyalkylierten Lactamen in hohen Umsätzen und hohen Reinheiten aus einfachen Edukten herstellbar sind. Die Synthese sollte zu Produkten mit einer niedrigen Farbzahl und hoher Reinheit führen. Insbesondere soll der Anteil von Michael-Addukten zurückgedrängt werden. Ein notwendiger Katalysator soll ferner einfach abtrennbar sein und keine weiteren Nachbehandlungen, z.B. in Form von Aufarbeitungsschritten, des Reaktionsproduktes zur Folge haben.

Die Aufgabe wurde gelöst durch ein Verfahren zur Herstellung von (Meth)acrylaten von N-hydroxyalkylierten Lactamen, in dem man ringförmige N-hydroxyalkylierte Lactame (C), 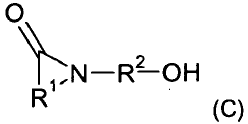 worin
R¹ C₁-C₅-Alkylen oder durch ein- oder mehrere Sauerstoff- und/oder Schwefelatome und/oder ein oder mehrere substituierte oder unsubstituierte Iminogruppen und/oder durch eine oder mehrere Cycloalkyl-, -(CO)-, -O(CO)O-, -(NH)(CO)O-, -O(CO)(NH)-, -O(CO)- oder -(CO)O-Gruppen unterbrochenes C₂-C₂₀-Alkylen, wobei die genannten Reste jeweils durch Aryl, Alkyl, Aryloxy, Alkyloxy, Heteroatome und/oder Heterocyclen substituiert sein können
mit der Maßgabe, daß R¹ kein anderes Atom als ein Kohlenstoffatom direkt benachbart zur Lactam-Carbonylgruppe aufweisen darf,
R² C₁-C₂₀-Alkylen, C₅-C₁₂-Cycloakylen, C₆-C₁₂-Arylen oder durch ein- oder mehrere Sauerstoff- und/oder Schwefelatome und/oder ein oder mehrere substituierte oder unsubstituierte Iminogruppen und/oder durch eine oder mehrere Cycloalkyl-, -(CO)-, -O(CO)O-, -(NH)(CO)O-, -O(CO)(NH)-, -O(CO)- oder -(CO)O-Gruppen unterbrochenes C₂-C₂₀-Alkylen, wobei die genannten Reste jeweils durch Aryl, Alkyl, Aryloxy, Alkyloxy, Heteroatome und/oder Heterocyclen substituiert sein können, oder
R²-OH für eine Gruppe der Formel -[Xᵢ]ₖ-H,
- k: für eine Zahl von 1 bis 50 und
- Xᵢ: für jedes i = 1 bis k unabhängig voneinander ausgewählt sein kann aus der Gruppe -CH₂-CH₂-O-, -CH₂-CH₂-N(H)-, -CH₂-CH₂-CH₂-N(H)-, -CH₂-CH(NH₂)-, -CH₂-CH(NHCHO)-, -CH₂-CH(CH₃)-O-, -CH(CH₃)-CH₂-O- -CH₂-C(CH₃)₂-O--C(CH₃)₂-CH₂-O-, -CH₂-CH₂-CH₂-O-, -CH₂-CH₂-CH₂- CH₂-O-, -CH₂-CHVin-O-, -CHVin-CH₂-O-, -CH₂-CHPh-O- und -CHPh-CH₂-O worin Ph für Phenyl und Vin für Viny steht,
bedeuten,
in Gegenwart mindestens eines heterogenen anorganischen Salzes (S) mit (Meth)acrylsäure verestert oder mit mindestens einem (Meth)acrylsäureester (D)
umestert.

Mit Hilfe des erfindungsgemäßen Verfahrens ist die Herstellung von (Meth)acrylaten von N-hydroxyalkylierten Lactamen mit mindestens einem der folgenden Vorteile möglich:
- hohe Ausbeute,
- gute Farbzahlen und
- keine Waschschritte zur Aufreinigung des Reaktionsgemischs erforderlich.

(Meth)acrylsäure steht in dieser Schrift für Methacrylsäure und Acrylsäure, bevorzugt für Methacrylsäure.

In den obigen Definitionen bedeuten
gegebenenfalls durch Aryl, Alkyl, Aryloxy, Alkyloxy, Heteroatome und/oder Heterocyclen substituiertes C₁-C₂₀-Alkylen beispielsweise Methylen, 1,2-Ethylen, 1,2-Propylen, 1,3-Propylen, 1,4-Butylen, 1,6-Hexylen, 2-Methyl-1,3-Propylen, 2-Ethyl-1,3-Propylen, 2,2-Dimethyl-1,3-Propylen, 2,2-Dimethyl-1,4-butylen,
gegebenenfalls durch Aryl, Alkyl, Aryloxy, Alkyloxy, Heteroatome und/oder Heterocyclen substituiertes C₅-C₁₂-Cycloalkylen beispielsweise Cyclopropylen, Cyclopentylen, Cyclohexylen, Cyclooctylen, Cyclododecylen,
gegebenenfalls durch Aryl, Alkyl, Aryloxy, Alkyloxy, Heteroatome und/oder Heterocyclen substituiertes, durch ein- oder mehrere Sauerstoff- und/oder Schwefelatome und/oder ein oder mehrere substituierte oder unsubstituierte Iminogruppen und/oder durch eine oder mehrere Cycloalkyl-, -(CO)-, -O(CO)O-, -(NH)(CO)O-, -O(CO)(NH)-, -O(CO)- oder -(CO)O-Gruppen unterbrochenes C₁-C₂₀-Alkylen beispielsweise 1-Oxa-1,3-propylen, 1,4-Dioxa-1,6-hexylen, 1,4,7-Trioxa-1,9-nonylen, 1-Oxa-1,4-butylen, 1,5-Dioxa-1,8-octylen, 1-Oxa-1,5-pentylen, 1-Oxa-1,7-heptylen, 1,6-Dioxa-1,10-decylen, 1-Oxa-3-methyl-1,3-propylen, 1-Oxa-3-methyl-1,4-butylen, 1-Oxa-3,3-dimethyl-1,4-butylen, 1-Oxa-3,3-dimethyl-1,5-pentylen, 1,4-Dioxa-3,6-dimethyl-1,6-hexylen, 1-Oxa-2-methyl-1,3-propylen, 1,4-Dioxa-2,5-dimethyl-1,6-hexylen, 1-Oxa-1,5-pent-3-enylen, 1-Oxa-1,5-pent-3-inylen, 1,1-, 1,2-, 1,3- oder 1,4-Cyclohexylen, 1,2- oder 1,3-Cyclopentylen, 1,2-, 1,3- oder 1,4-Phenylen, 4,4'-Biphenylen, 1,4-Diaza-1,4-butylen, 1-Aza-1,3-propylen, 1,4,7-Triaza-1,7-heptylen, 1,4-Diaza-1,6-hexylen, 1,4-Diaza-7-oxa-1,7-heptylen, 4,7-Diaza-1-oxa-1,7-heptylen, 4-Aza-1-oxa-1,6-hexylen, 1-Aza-4-oxa-1,4-butylen, 1-Aza-1,3-propylen, 4-Aza-1-oxa-1,4-butylen, 4-Aza-1,7-dioxa-1,7-heptylen, 4-Aza-1-oxa-4-methyl-1,6-hexylen, 4-Aza-1,7-dioxa-4-methyl-1,7-heptylen, 4-Aza-1,7-dioxa-4-(2'-hydroxyethyl)-1,7-heptylen, 4-Aza-1-oxa-(2'-hydroxyethyl)-1,6-hexylen oder 1,4-Piperazinylen und
gegebenenfalls durch Aryl, Alkyl, Aryloxy, Alkyloxy, Heteroatome und/oder Heterocyclen substituiertes C₆-C₁₂-Arylen beispielsweise 1,2-, 1,3- oder 1,4-Phenylen, 4,4'-Biphenylen, Toluylen oder Xylylen.

Beispiele für R¹ sind 1,2-Ethylen, 1,2-Propylen, 1,1-Dimethyl-1,2-ethylen, 1-Hydroxymethyl-1,2-ethylen, 2-Hydroxy-1,3-propylen, 1,3-Propylen, 1,4-Butylen, 1,5-Pentylen, 2-Methyl-1,3-Propylen, 2-Ethyl-1,3-Propylen, 2,2-Dimethyl-1,3-Propylen und 2,2-Dimethyl-1,4-butylen, bevorzugt sind 1,4-Butylen, 1,5-Pentylen und 1,3-Propylen, besonders bevorzugt ist 1,3-Propylen.

Beispiele für R² sind 1,2-Ethylen, 1,2-Propylen, 1,1-Dimethyl-1,2-ethylen, 1-Hydroxymethyl-1,2-ethylen, 2-Hydroxy-1,3-propylen, 1,3-Propylen, 1,4-Butylen, 1,6-Hexylen, 2-Methyl-1,3-Propylen, 2-Ethyl-1,3-Propylen, 2,2-Dimethyl-1,3-Propylen und 2,2-Dimethyl-1,4-butylen, 1,2-Cyclopentylen, 1,3-Cyclopentylen, 1,2-Cyclohexylen,1,3-Cyclohexylen, ortho-Phenylen, 3-Oxa-1,5-pentylen, 3,6-Dioxa-1,8-octylen und 3,6,8-Trioxa-1,8,11-undecylen, bevorzugt sind 1,2-Ethylen, 1,2-Propylen, 1,3-Propylen, besonders bevorzugt sind 1,2-Ethylen und 1,2-Propylen und ganz besonders bevorzugt ist 1,2-Ethylen.

Bevorzugte Individuen (C) sind N-(2-Hydroxyethyl)-pyrrolidon, N-(2-Hydroxypropyl)-pyrrolidon, N-(2'-(2-Hydroxyethoxy)-ethyl)-pyrrolidon, N-(2-Hydroxyethyl)-caprolactam, N-(2-Hydroxypropyl)-caprolactam sowie N-(2'-(2-Hydroxyethoxy)-ethyl)-caprolactam, bevorzugt sind N-(2-Hydroxyethyl)-pyrrolidon und N-(2-Hydroxypropyl)-pyrrolidon, besonders bevorzugt ist N-(2-Hydroxyethyl)-pyrrolidon.

Die Veresterung des Alkohols (C) mit (Meth)acrylsäure oder bevorzugt die Umesterung des Alkohols (C) mit mindestens einem, bevorzugt genau einem (Meth)acrylsäureester (D) erfolgt erfindungsgemäß in Anwesenheit mindestens eines heterogenen anorganischen Salzes (S).

Verbindungen (D) können Ester von (Meth)acrylsäure mit einem gesättigten Alkohol sein, bevorzugt gesättigte C₁ - C₁₀-Alkylester der (Meth)acrylsäure, besonders bevorzugt gesättigte C₁ - C₄-Alkylester der (Meth)acrylsäure.
Gesättigt bedeutet im Rahmen dieser Schrift Verbindungen ohne C-C-Mehrfachbindungen (außer selbstverständlich die C=C-Doppelbindung in den (Meth)acryleinheiten).

Beispiele für Verbindungen (D) sind (Meth)acrylsäuremethyl-, -ethyl-, -n-butyl-, -iso-butyl-, -tert-butyl-, n-octyl- und -2-Ethylhexylester, 1,2-Ethylenglycoldi- und -mono(meth)acrylat, 1,4-Butandioldi- und -mono(meth)acrylat, 1,6-Hexandioldi- und -mono(meth)acrylat, Trimethylolpropantri(meth)acrylat und Pentaerythrit-tetra(meth)acrylat.

Besonders bevorzugt sind (Meth)acrylsäuremethyl-, -ethyl-, -n-butyl- und -2-Ethyl-hexylester, ganz besonders bevorzugt (Meth)acrylsäuremethyl-, -ethyl- und -n-butylester, insbesondere (Meth)acrylsäuremethyl- und -ethylester und speziell (Meth)acrylsäuremethylester.

Erfindungsgemäß einsetzbare anorganischen Salzen (S) sind solche, die einen pK_{B}-Wert von nicht mehr als 7,0, bevorzugt von nicht mehr als 6,1 und besonders bevorzugt von nicht mehr als 4,0 aufweisen. Gleichzeitig sollte der pK_{B}-Wert 1,0 nicht unterschreiten, bevorzugt nicht weniger als 1,5 betragen und besonders bevorzugt nicht weniger als 1,6.

Heterogene Katalysatoren sind im Rahmen dieser Schrift erfindungsgemäß solche, die eine Löslichkeit im Reaktionsmedium bei 25°C von nicht mehr als 1 g/l aufweisen, bevorzugt von nicht mehr als 0,5 g/l und besonders bevorzugt von nicht mehr als 0,25 g/l.

Das anorganische Salz weist bevorzugt mindestens ein Anion auf ausgewählt aus der Gruppe bestehend aus Carbonat (CO₃²⁻), Hydrogencarbonat (HCO₃⁻), Phosphat (PO₄³⁻), Hydrogenphosphat (HPO₄²⁻), Dihydrogenphosphat (H₂PO₄⁻), Sulfat (SO₄²⁻), Hydrogensulfat (HSO₄⁻), Hydrogensulfit (HSO₃⁻) und Carboxylat (R⁶-COO⁻), worin R⁶ C₁- C₁₈-Alkyl oder gegebenenfalls durch ein oder mehrere Sauerstoff- und/oder Schwefelatome und/oder ein oder mehrere substituierte oder unsubstituierte Iminogruppen unterbrochenes C₂ - C₁₈-Alkyl oder C₆- C₁₂-Aryl bedeutet.

Bevorzugt sind Carbonat und Phosphat.

Unter Phosphat sind auch die Kondensationsprodukte zu verstehen, wie beispielsweise Diphosphate, Triphosphate und Polyphosphate.

Das anorganische Salz weist bevorzugt mindestens ein Kation auf ausgewählt aus der Gruppe bestehend aus Alkalimetallen, Erdalkalimetallen, Ammonium, Cer, Eisen, Mangan, Chrom, Molybdän, Kobalt, Nickel oder Zink.
Bevorzugt sind Alkalimetalle und besonders bevorzugt sind Lithium, Natrium oder Kalium.

Besonders bevorzugte anorganische Salze sind Li₃PO₄, K₃PO₄, Na₃PO₄ und K₂CO₃, sowie deren Hydrate, ganz besonders bevorzugt sind K₂CO₃ und K₃PO₄.

K₃PO₄ kann erfindungsgemäß in wasserfreier Form eingesetzt werden sowie als Tri-, Hepta- oder Nonahydrat.

Die durch ein anorganisches Salz katalysierte Ver- oder Umesterung erfolgt im Allgemeinen bei 30 bis 140°C, bevorzugt bei 30 bis 100°C, besonders bevorzugt bei 40 bis 90°C und ganz besonders bevorzugt bei 50 bis 80 °C.

Gegebenenfalls kann die Reaktion unter leichtem Vakuum von beispielsweise 150 hPa bis Normaldruck, bevorzugt 200 bis 600 hPa und besonders bevorzugt 200 bis 400 hPa durchgeführt werden, wenn das bei der Veresterung freigesetzte Wasser oder der bei der Umesterung entstehende niedrigsiedende Alkohol, gegebenenfalls als Azeotrop, abdestilliert werden soll.

Das molare Verhältnis zwischen (Meth)Acrylsäure oder (Meth)Acrylsäureester und Alkohol (C) beträgt bei der durch ein anorganisches Salz katalysierten Ver- oder Umesterung in der Regel 1-6: 1 mol/mol, bevorzugt 1 - 5,5 : 1 mol/mol und besonders bevorzugt 1 - 5,0 : 1 mol/mol.

Die Reaktionszeit bei der durch ein anorganisches Salz katalysierten Ver- oder Umesterung beträgt in der Regel 45 min bis 18 Stunden, bevorzugt 1,5 Stunden bis 12 Stunden und besonders bevorzugt 2 bis 8 Stunden.

Der Gehalt an anorganischen Salzen im Reaktionsmedium liegt in der Regel im Bereich von etwa 0,01 bis 5 mol%, bevorzugt 0,1 - 1,8 und besonders bevorzugt 1 - 2,5 mol% bezogen auf die Summe der eingesetzten Komponenten (C).

Bei der Ver- oder Umesterung sind Polymerisationsinhibitoren (s.u.) zwingend erforderlich.

Die Anwesenheit von sauerstoffhaltigen Gasen (s.u.) während der durch ein anorganisches Salz katalysierten Reaktion ist bevorzugt.

Bei der durch ein anorganisches Salz katalysierten Ver- oder Umesterung werden in der Regel die Produkte mit einer Farbzahl unter 500 APHA, bevorzugt unter 200 und besonders bevorzugt unter 150 (gemäß DIN ISO 6271) erhalten.

Die Reaktion kann in organischen Lösungsmitteln oder deren Gemischen oder ohne Zusatz von Lösungsmitteln ablaufen. Die Ansätze sind in der Regel weitgehend wasserfrei (d.h. unter 10, bevorzugt unter 5, besonders bevorzugt unter 1 und ganz besonders bevorzugt unter 0,5 Gew% Wassergehalt). Ferner sind die Ansätze weitgehend frei von primären und sekundären Alkoholen, d.h. unter 10, bevorzugt unter 5, besonders bevorzugt unter 1 und ganz besonders bevorzugt unter 0,5 Gew% Alkoholgehalt. Geeignete organische Lösungsmittel sind solche für diese Zwecke bekannten, beispielsweise tertiäre Monoole, wie C₃-C₆-Alkohole, bevorzugt tert-Butanol, tert-Amylalkohol, Pyridin, Poly-C₁-C₄-alkylenglykoldi-C₁-C₄-alkylether, bevorzugt Polyethylenglycoldi-C₁-C₄-alkylether, wie z.B. 1,2-Dimethoxyethan, Diethylenglycoldimethylether, Polyethylenglycoldimethylether 500, C₁-C₄-Alkylencarbonate, insbesondere Propylencarbonat, C₃-C₆-Alkylessigsäureester, insbesondere tert.-Butyl-essigsäureester, THF, Toluol, 1,3-Dioxolan, Aceton, iso-Butyl-methylketon, Ethylmethylketon, 1,4-Dioxan, tert-Butylmethylether, Cyclohexan, Methylcyclohexan, Toluol, Hexan, Dimethoxymethan, 1,1-Dimethoxyethan, Acetonitril, sowie deren ein- oder mehrphasige Mischungen.

In einer besonders bevorzugten Ausführungsform der Umesterung wird die Reaktion in dem als Edukt eingesetzten (Meth)Acrylsäureester durchgeführt. Ganz besonders bevorzugt ist die Durchführung der Reaktion in der Weise, daß das Produkt (C) nach Beendigung der Reaktion als etwa 10-80 Gew%ige Lösung in dem als Edukt eingesetzten (Meth)Acrylsäureester anfällt, insbesondere als 20 bis 50 Gew%ige Lösung.

Die Edukte liegen entweder gelöst, als Feststoffe suspendiert oder in Emulsion im Reaktionsmedium vor.

Die Reaktion kann kontinuierlich, beispielsweise in einem Rohrreaktor oder in einer Rührreaktorkaskade, oder diskontinuierlich erfolgen.

Die Umsetzung kann in allen für eine solche Umsetzung geeigneten Reaktoren durchgeführt werden. Solche Reaktoren sind dem Fachmann bekannt. Bevorzugt erfolgt die Umsetzung in einem Rührkesselreaktor oder einem Festbettreaktor.

Zur Durchmischung des Reaktionsansatzes können beliebige Verfahren eingesetzt werden. Spezielle Rührvorrichtungen sind nicht erforderlich. Die Durchmischung kann beispielsweise durch Einspeisen eines Gases, bevorzugt eines sauerstoffhaltigen Gases (siehe unten) erfolgen. Das Reaktionsmedium kann ein- oder mehrphasig sein und die Reaktanden werden darin gelöst, suspendiert oder emulgiert. Die Temperatur wird während der Reaktion auf den gewünschten Wert eingestellt und kann, falls gewünscht, während des Reaktionsverlauf erhöht oder verringert werden.

Die Entfernung von Wasser im Falle einer Veresterung oder Alkoholen, die bei einer Umesterung aus den Alkyl(meth)acrylaten freigesetzt werden, erfolgt kontinuierlich oder schrittweise in an sich bekannter Weise, z.B. durch Vakuum, azeotrope Entfernung, Strippen, Absorption, Pervaporation und Diffusion über Membranen oder Extraktion.

Das Strippen kann beispielsweise durch Durchleiten eines sauerstoffhaltigen Gases, bevorzugt eines Luft oder Luft-Stickstoff-Gemisches, durch das Reaktionsgemisch erfolgen, gegebenenfalls zusätzlich zu einer Destillation.
Zur Absorption eignen sich vorzugsweise Molekularsiebe oder Zeolithe (Porengröße z.B. im Bereich von etwa 3-10 Angström), eine Abtrennung durch Destillation oder mit Hilfe geeigneter semipermeabler Membranen.

Es ist aber auch möglich, das abgetrennte Gemisch aus Alkyl(meth)acrylat und dem diesem zugrundeliegenden Alkohol, das häufig ein Azeotrop bildet, direkt in eine Anlage zur Herstellung des Alkyl(meth)acrylats zuzuführen, um es dort in einer Veresterung mit (Meth)acrylsäure wiederzuverwerten.

Nach Beendigung der Reaktion kann man das erhaltene Reaktionsgemisch ohne weitere Aufreinigung verwenden oder es erforderlichenfalls in einem weiteren Schritt aufreinigen.

In der Regel wird im Aufreinigungsschritt lediglich der eingesetzte heterogen Katalysator vom Reaktionsgemisch abgetrennt und das Reaktionsprodukt vom gegebenenfalls verwendeten organischen Lösungsmittel abgetrennt.

Eine Abtrennung vom heterogenen Katalysator erfolgt in der Regel durch Filtration, Elektrofiltration, Absorption, Zentrifugation oder Dekantieren. Der abgetrennte heterogene Katalysator kann anschließend für weitere Reaktionen eingesetzt werden.

Die Abtrennung vom organischen Lösungsmittel erfolgt in der Regel durch Destillation, Rektifikation oder bei festen Reaktionsprodukten durch Filtration.

Bevorzugt werden im Aufreinigungsschritt jedoch lediglich der heterogene Katalysator und das gegebenenfalls eingesetzte Lösungsmittel abgetrennt.

Das gegebenenfalls aufgereinigte Reaktionsgemisch wird bevorzugt einer Destillation unterworfen, in der das (Meth)acrylat der N-hydroxyalkylierten Lactame destillativ von unumgesetzer (Meth)Acrylsäure oder unumgesetztem (Meth)Acrylsäureester (D) sowie gegebenenfalls gebildeten Nebenprodukten getrennt wird.

Bei den Destillationseinheiten handelt es sich zumeist um Rektifikationskolonnen üblicher Bauart mit Umlaufverdampfer und Kondensator. Der Zulauf erfolgt in bevorzugt den Sumpfbereich, die Sumpftemperatur beträgt hier beispielsweise 100-180 °C, bevorzugt 110-170 und besonders bevorzugt 125-155 °C, die Kopftemperatur bevorzugt 140 -145°C und der Kopfdruck 3 - 20, bevorzugt 3 bis 5 mbar. Selbstverständlich kann der Fachmann auch andere Temperatur- und Druckbereiche ermitteln, in denen die jeweiligen (Meth)acrylate der N-hydroxyalkylierten Lactame destillativ gereinigt werden können. Wesentlich ist dabei eine Trennung des Wunschproduktes von Edukten und Nebenprodukten unter Bedingungen, bei denen das Wunschprodukt möglichst keiner Abbaureaktion ausgesetzt ist.
Die Destillationseinheit weist in der Regel 5 bis 50 theoretische Böden auf.

Die Destillationseinheiten sind von an sich bekannter Bauart und weisen die üblichen Einbauten auf. Als Kolonneneinbauten kommen prinzipiell alle gängigen Einbauten in Betracht, beispielsweise Böden, Packungen und/oder Schüttungen. Von den Böden sind Glockenböden, Siebböden, Ventilböden, Thormannböden und/oder Dual-Flow-Böden bevorzugt, von den Schüttungen sind solche mit Ringen, Wendeln, Sattelkörpern, Raschig-, Intos- oder Pall-Ringen, Barrel- oder Intalox-Sätteln, Top-Pak etc. oder Geflechten bevorzugt.

Bevorzugt wird das Wunschprodukt diskontinuierlich destilliert, wobei zunächst Leichtsieder aus dem Reaktionsgemisch entfernt werden, zumeist Lösungsmittel oder unumgesetzte (Meth)Acrylsäure oder (Meth)Acrylsäureester (D). Nach Abtrennung dieser Leichtsieder wird die Destillationstemperatur erhöht und/oder das Vakuum verringert und das Wunschprodukt abdestilliert.

Der verbleibende Destillationsrückstand wird zumeist verworfen.

Aufgrund der Reaktionsbedingungen wird die Bildung von Nebenprodukten in der Reaktion vermieden, die andernfalls zum Beispiel von chemischen Katalysatoren stammen können oder durch unerwünschte radikalische Polymerisation des eingesetzten (Meth)acrylats, die sonst nur durch Zugabe von Stabilisatoren verhindert werden kann. Bei der erfindungsgemäßen Reaktionsführung kann der Reaktionsmischung über den ohnehin in der (Meth)acrylverbindung enthaltenen Lagerstabilisator hinaus zusätzlicher Stabilisator zugegeben werden, beispielsweise Hydrochinonmonomethylether, Phenothiazin, Phenole, wie z.B. 2-tert.-Butyl-4-methylphenol, 6-tert.-Butyl-2,4-dimethylphenol, aromatische Diamine, wie beispielsweise N,N-Di-sek.-butyl-p-phenylendiamin oder N-Oxyle, wie 4-Hydroxy-2,2,6,6-tetramethyl-piperidin-N-oxyl, 4-Oxo-2,2,6,6-tetramethyl-piperidin-N-oxyl oder Uvinul® 4040P der BASF Aktiengesellschaft, beispielsweise in Mengen von 10, bevorzugt von 50 bis 2000 ppm.

Vorteilhaft wird die Ver- oder Umesterung in Gegenwart eines sauerstoffhaltigen Gases, bevorzugt Luft oder Luft-Stickstoff-Gemische, durchgeführt.

Der erfindungsgemäß verwendete heterogene Katalysator kann durch dessen geringe Löslichkeit unproblematisch vom Endprodukt entfernt werden. Dadurch reicht zur Aufarbeitung des Reaktionsproduktes in der Regel eine einfache Filtration oder Dekantierung, auf aufwendige Wäschen, Neutralisationen, Destillationen und dergleichen zur Abtrennung des Katalysators kann in der Regel verzichtet werden.

Die erfindungsgemäß eingesetzten Katalysatoren zeigen eine lediglich geringe Tendenz zu Nebenreaktionen. Die anorganischen Salze sind zumeist ausreichend basisch um eine Umesterung zu katalysieren aber nicht zu basisch, um Nebenreaktionen, wie beispielsweise Michael-Reaktionen, in größerem Umfang zu katalysieren.

Des weiteren ist die Reaktion unter den erfindungsgemäßen Reaktionsbedingungen sehr selektiv, man findet in der Regel weniger als 10 %, bevorzugt weniger als 5 % Nebenprodukte (bezogen auf den Umsatz).

Die erfindungsgemäß hergestellten (Meth)acrylate von N-hydroxyalkylierten Lactame finden Anwendung beispielsweise als Monomere oder Comonomere in der Herstellung von Poly(meth)acrylaten und Dispersionen, beispielsweise Acryldispersionen, als Reaktivverdünner, beispielsweise in strahlungshärtbaren Beschichtungsmassen oder in Farben, bevorzugt in Außenfarben, sowie in Dispersionen für Anwendung im Papierbereich.

Die folgenden Beispiele sollen die Eigenschaften der Erfindung erläutern, ohne sie aber einzuschränken.

### Beispiele

Als "Teile" seien in dieser Schrift, wenn nicht anders angegeben, "Gewichtsteile" verstanden.

### Beispiele 1 bis 4

In einer Umesterungsapparatur (1 L-4-Halskolben mit Vigreux-Kolonne und Flüssigkeitsverteiler) wurde die Umesterung von Methylmethacrylat (MMA) mit Hydroxyethylpyrrolidon (HEP) bei einer Badtemperatur von 100°C unter Vakuum (ca. 400-700 mbar) über 4-5 Stunden durchgeführt. Dabei wurde Hydroxyethylpyrrolidon als Flüssigkeit vorgelegt, mit Methylmethacrylat verdünnt (Molverhältnis Methylmethacrylat : Hydroxyethylpyrrolidon = 5,6 : 1) und anschließend durch Zugabe des Katalysators die Reaktion gestartet. Das entstehende Methanol wurde anschließend als Azeotrop mit Methylmethacrylat aus der Reaktionsmischung entfernt.

Zur Stabilisierung von Methylmethacrylat bzw. der im Laufe der Reaktion entstehenden Methacrylate wurden Stabilisatoren (50 ppm Phenothiazin und 500 ppm Hydrochinonmonomethylether) zugesetzt und Luft eingeleitet. Verschiedene Katalysatoren wurden getestet (Einsatz jeweils von 0,8 Mol-% bezogen auf Hydroxyethylpyrrolidon).

Nach Ende der Reaktionszeit wurde der zurückbleibende Sumpf gaschromatographisch analysiert (Angaben in Flächenprozent):

**Tabelle 1: Umsatz von Hydroxyethylpyrrolidon mit verschiedenen Katalysatoren**

| Beispiel | Katalysator | Methanol [FI-%] | Methylmethacrylat [FI-%] | Hydroxyethylpyrrolidon [FI-%] | Wertprodukt [FI-%] |
|---|---|---|---|---|---|
| 1 | K₃PO₄ | 0,1 | 25,3 | 5,2 | 66,2 |
| 2 | K₂CO₃ | 0,1 | 2,6 | 16,9 | 78,8 |
| 3 (Vergleich) | NaOCH₃ | 0,1 | 42,2 | 13,9 | 42,4 |
| 4 (Vergleich) | Ti(OiPr)₄ | - | 21,7 | 75,1 | 2,6 |

Man erkennt, daß mit den erfindungsgemäßen Katalysatoren ein deutlich höherer Anteil an Wertprodukt durch eine Umesterung erhalten werden konnte, als mit Natriummethanolat oder Titantetraisopropanolat.

### Beispiel 5

In einem 750ml Reaktor mit Oldershaw-Kolonne und Flüssigkeitsverteiler wurden bei einer Rührgeschwindigkeit (Anker-Rührer) von 400 U/min und einer Lufteinleitung von 1,5 l/h 280 mg Hydrochinonmonomethylether (350 ppm), 600 g (6,0 mol) Methylmethacrylat (MMA) und 194 g (1,5 mol) N-Hydroxyethyl-Pyrrolidon vorgelegt und gerührt. Anschließend wurden 6,37 g (30 mmol; 2,0 Mol-% bezogen auf N-Hydroxyethyl-Pyrrolidon) wasserfreies Kaliumphosphat zugegeben, das Vakuum eingestellt (300 mbar) und die Suspension erhitzt (die Manteltemperatur wurde mittels Thermostaten auf 120 °C eingestellt). Nach ca. 20 Minuten begann die Suspension zu sieden (t = 0 min). Während der Reaktion wurde kontinuierlich Destillat entfernt (Rücklaufverhältnis Rücklauf:Ablauf betrug 25:1) und die Temperatur im Sumpf stieg von ca. 63 °C auf 79 °C an. Nach 300 min wurde die Reaktion beendet und das Vakuum aufgehoben. Die Suspension wurde abgekühlt und anschließend über eine Druckfilternutsche filtriert (30 ml Methylmethacrylat wurden zum Spülen vom Rückstand nachgegeben). Man erhielt 492 g Rohprodukt, welches mit 200 ppm N,N-Di-sek.-butyl-p-phenylendiamin (98 mg) versetzt und unter Lufteinleitung destilliert wurde. Dabei wurde zunächst das überschüssige Methylmethacrylat entfernt und anschließend das Wertprodukt erhalten (130°C, 1,3 mbar). Man erhielt 273g (92%) N-Hydroxyethyl-Pyrrolidon-Methacrylat (N-(2-(Methacryloyl)-ethyl)-pyrrolidon) in hoher Reinheit (GC-Analyse: 99,6%). Die Farbzahl betrug 50 (APHA-Farbzahl).

Somit erhält man bei der Umesterung mit einem erfindungsgemäßen Katalysator eine höhere Ausbeute als bei der sauren Veresterung gemäß WO 03/6568.

### Beispiel 6

In der in Beispiel 5 beschriebenen Apparatur wurden 280 mg Hydrochinonmonomethylether (350 ppm), 600 g (6,0 mol) Methylmethacrylat (MMA) und 194 g (1,5 mol) N-Hydroxyethyl-Pyrrolidon vorgelegt und gerührt. Anschließend wurden 4,15 g (30 mmol; 2,0 Mol-% bezogen auf N-Hydroxyethyl-Pyrrolidon) Kaliumcarbonat zugegeben, das Vakuum eingestellt (300 mbar) und die Suspension erhitzt (die Manteltemperatur wurde mittels Thermostaten auf 120 °C eingestellt). Nach ca. 20 Minuten begann die Suspension zu sieden (t = 0 min). Während der Reaktion wurde kontinuierlich Destillat entfernt und die Temperatur im Sumpf stieg von ca. 63°C auf 79 °C an. Nach 300 min wurde die Reaktion beendet und das Vakuum aufgehoben. Die Suspension wurde abgekühlt und anschließend über eine Druckfilternutsche filtriert (30 ml MMA wurden zum Spülen vom Rückstand nachgegeben).

Man erhielt 438 g Rohprodukt. Zunächst wurde das überschüssige MMA entfernt. Anschließend wurden dem von MMA befreiten Rohprodukt 400 ppm N,N-Di-sek.-butyl-p-phenylendiamin (106 mg) zugeben und unter Lufteinleitung destilliert. Das Wertprodukt wurde erhalten (147 °C, 3,6 mbar). Man erhielt 238g (81%) N-Hydroxyethyl-Pyrrolidon-Methacrylat (N-(2-(Methacryloyl)-ethyl)-pyrrolidon) in hoher Reinheit (GC-Analyse: 99,7%). Die Farbzahl betrug ca. 60 (APHA-Farbzahl).

### Beispiel 7

In der in Beispiel 5 beschriebenen Apparatur wurden 280 mg Hydrochinonmonomethylether (350 ppm), 600 g (6,0 mol) Methylmethacrylat und 194 g (1,5 mol) N-Hydroxyethylpyrrolidon (APHA-Farbzahl = 117) vorgelegt und gerührt. Anschließend wurden 4,15 g (30 mmol; 2,0 Mol-% bezogen auf N-Hydroxyethylpyrrolidon) Kaliumcarbonat zugegeben, das Vakuum eingestellt (300 mbar) und die Suspension erhitzt (die Manteltemperatur wurde mittels Thermostaten auf 120°C eingestellt). Nach ca. 15 Minuten begann die Suspension zu sieden (t = 0 min). Während der Reaktion wurde kontinuierlich Destillat (Azeotrop aus MMA und Methanol) entfernt. Nach 150 min wurde die Temperatur im Sumpf auf 60 °C eingestellt und das überschüssige MMA im Vakuum entfernt. Anschließend wurde das Vakuum aufgehoben, die Suspension abgekühlt und über eine Druckfilternutsche filtriert. Man erhielt 289 g Produkt (Ausbeute = 98%) als leicht gelbliche Flüssigkeit (APHA-Farbzahl = 121) mit folgender Zusammensetzung (GC-Analyse, Angaben in %):

| Produkt* | HEP** | MMA | Nebenprodukte*** | Farbzahl (APHA) |
|---|---|---|---|---|
| 96,5 | 0,4 | 1,0 | 2,1 | 121 |

| | | | | |
|---|---|---|---|---|
| * N-Hydroxyethylpyrrolidon-Methacrylat ** N-Hydroxyethylpyrrolidon ***Summe Nebenprodukte | | | | |

Das Produkt wurde bezüglich des Gehaltes an Kalium untersucht. Es wurde ein Gehalt von < 0,001g/100g gefunden.

## Patentansprüche

1. Verfahren zur Herstellung von (Meth)acrylaten von N-hydroxyalkylierten Lactamen, in dem man ringförmige N-hydroxyalkylierte Lactame (C), 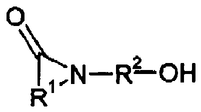 worin
R¹ C₁-C₅-Alkylen oder durch ein- oder mehrere Sauerstoff- und/oder Schwefelatome und/oder ein oder mehrere substituierte oder unsubstituierte Iminogruppen und/oder durch eine oder mehrere Cycloalkyl-, -(CO)-, -O(CO)O-, -(NH)(CO)O-, -O(CO)(NH)-, -O(CO)- oder -(CO)O-Gruppen unterbrochenes C₂-C₂₀-Alkylen, wobei die genannten Reste jeweils durch Aryl, Alkyl, Aryloxy, Alkyloxy, Heteroatome und/oder Heterocyclen substituiert sein können
mit der Maßgabe, daß R¹ kein anderes Atom als ein Kohlenstoffatom direkt benachbart zur Lactam-Carbonylgruppe aufweisen darf,
R² C₁-C₂₀-Alkylen, C₅-C₁₂-Cycloakylen, C₆-C₁₂-Arylen oder durch ein- oder mehrere Sauerstoff- und/oder Schwefelatome und/oder ein oder mehrere substituierte oder unsubstituierte Iminogruppen und/oder durch eine oder mehrere Cycloalkyl-, -(CO)-, -O(CO)O-, -(NH)(CO)O-, -O(CO)(NH)-, -O(CO)- oder -(CO)O-Gruppen unterbrochenes C₂-C₂₀-Alkylen, wobei die genannten Reste jeweils durch Aryl, Alkyl, Aryloxy, Alkyloxy, Heteroatome und/oder Heterocyclen substituiert sein können, oder
R²-OH für eine Gruppe der Formel -[Xᵢ]ₖ-H,
k für eine Zahl von 1 bis 50 und
Xᵢ für jedes i = 1 bis k unabhängig voneinander ausgewählt sein kann aus der Gruppe -CH₂-CH₂-O-, -CH₂-CH₂-N(H)-, -CH₂-CH₂-CH₂-N(H)-, -CH₂-CH(NH₂)-, -CH₂-CH(NHCHO)-, -CH₂-CH(CH₃)-O-, -CH(CH₃)-CH₂-O-, -CH₂-C(CH₃)₂-O-, -C(CH₃)₂-CH₂-O-, -CH₂-CH₂-CH₂-O-, -CH₂-CH₂-CH₂- CH₂-O-, -CH₂-CHVin-O-, -CHVin-CH₂-O-, -CH₂-CHPh-O- und -CHPh-CH₂-O-, worin Ph für Phenyl und Vin für Vinyl steht,
bedeuten,
in Gegenwart mindestens eines heterogenen anorganischen Satzes (S) mit (Meth)acrylsäure verestert oder mit mindestens einem (Meth)acrylsäureester (D) umestert.

2. Verfahren gemäß Anspruch 1, **dadurch gekennzeichnet, daß** das mindestens eine anorganische Salz (S) einen pKₐ-Wert von nicht mehr als 7,0 und nicht weniger als 1,0, sowie eine Löslichkeit im Reaktionsmedium bei 25 °C von nicht mehr als 1 g/l aufweist.

3. Verfahren gemäß einem der vorstehenden Ansprüche, **dadurch gekennzeichnet, daß** das anorganische Salz mindestens ein Anion aufweist, ausgewählt aus der Gruppe bestehend aus Carbonat (CO₃²), Hydrogencarbonat (HCO₃⁻), Phosphat (PO₄³⁻), Hydrogenphosphat (HPO₄²⁻), Dihydrogenphosphat (H₂PO₄⁻), Sulfat (SO₄²⁻), Hydrogensulfat (HSO₄⁻), Sulfit (SO₃²⁻), Hydrogensulfit (HSO₃⁻) und Carboxylat (R⁶-COO⁻), worin R⁶ C₁ - C₁₆-Alkyl oder gegebenenfalls durch ein oder mehrere Sauerstoff- und/oder Schwefelatome und/oder ein oder mehrere substituierte oder unsubstituierte Iminogruppen unterbrochenes C₂ - C₁₈-Alkyl oder C₆ - C₁₂-Aryl bedeutet.

4. Verfahren gemäß einem der vorstehenden Ansprüche, **dadurch gekennzeichnet, daß** das anorganische Salz mindestens ein Kation aufweist, ausgewählt aus der Gruppe bestehend aus Akalimetallen, Erdalkalimetallen, Ammonium, Cer, Eisen, Mangan, Chrom, Molybdän, Kobalt, Nickel oder Zink.

5. Verfahren gemäß einem der vorstehenden Ansprüche, **dadurch gekennzeichnet, daß** das anorganische Salz ausgewählt ist aus der Gruppe bestehend aus Li₃PO₄, K₃PO₄, Na₃PO₄ und K₂CO₃, sowie deren Hydrate.

6. Verfahren gemäß einem der vorstehenden Ansprüche, **dadurch gekennzeichnet, daß** R² ausgewählt ist aus der Gruppe bestehend aus 1,2-Ethylen, 1,2-Propylen, 1,1-Dimethyl-1,2-ethylen, 1-Hydroxymethyl-1,2-ethylen, 2-Hydroxy-1,3-propylen, 1,3-Propylen, 1,4-Butylen, 1,6-Hexylen, 2-Methyl-1,3-Propylen, 2-Ethyl-1,3-Propylen, 2,2-Dimethyl-1,3-Propylen und 2,2-Dimethyl,1,4-butylen, 1,2-Cyclopentylen, 1,3-Cyclopentylen, 1,2-Cyclohexylen,1,3-Cyclohexylen, ortho-Phenylen, 3-Oxa-1,5-pentylen, 3,6-Dioxa-1,8-octylen und 3,6,8-Trioxa-1,8,11-undecylen.

7. Verfahren gemäß einem der vorstehenden Ansprüche, **dadurch gekennzeichnet, daß** R¹ ausgewählt ist aus der Gruppe bestehend aus 1,2-Ethylen, 1,2-Propylen, 1,1-Dimethyl-1,2-ethylen, 1-Hydroxymethyl-1,2-ethylen, 2-Hydroxy-1,3-propylen, 1,3-Propylen, 1,4-Butylen, 1,5-Pentylen, 2-Methyl-1,3-Propylen, 2-Ethyl-1,3-Propylen, 2,2-Dimethyl-1,3-Propylen und 2,2-Dimethyl-1,4-butylen.

8. Verfahren gemäß einem der vorstehenden Ansprüche, **dadurch gekennzeichnet, daß** (C) ausgewählt ist aus der Gruppe bestehend aus N-(2-Hydroxyethyl)-pyrrolidon, N-(2-Hydroxypropyl)-pyrrolidon, N-(2'-(2-Hydroxyethoxy)-ethyl)-pyrrolidon, N-(2-Hydroxyethyl)-caprolactam, N-(2-Hydroxypropyl)-caprolactam und N-(2'-(2-Hydroxyethoxy)-ethyl)-caprolactam.

## Claims

1. A process for preparing (meth)acrylates of N-hydroxyalkylated lactams, in which cyclic N-hydroxyalkylated lactams (C) 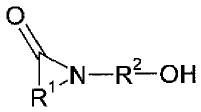 in which
R¹ is C₁-C₅-alkylene, or C₂-C₂₀-alkylene interrupted by one or more oxygen and/or sulfur atoms and/or one or more substituted or unsubstituted imino groups and/or by one or more cycloalkyl, -(CO)-, -O(CO)O-, -(NH)(CO)O-, -O(CO)(NH)-, -O(CO)- or -(CO)O- groups, where the radicals mentioned may each be substituted by aryl, alkyl, aryloxy, alkyloxy, heteroatoms and/or heterocycles,
with the proviso that R¹ must not have any atom other than a carbon atom directly adjacent to the lactam carbonyl group,
R² is C₁-C₂₀-alkylene, C₅-C₁₂₋cycloalkylene, C₆-C₁₂-arylene, or C₂-C₂₀-alkylene interrupted by one or more oxygen and/or sulfur atoms and/or one or more substituted or unsubstituted imino groups and/or by one or more cycloalkyl, -(CO)-, -O(CO)O-, -(NH)(CO)O-, -O(CO)(NH)-, -O(CO)- or -(CO)O- groups, where the radicals mentioned may each be substituted by aryl, alkyl, aryloxy, alkyloxy, heteroatoms and/or heterocycles, or
R²-OH is a group of the formula -[Xᵢ]ₖ-H,
k is from 1 to 50 and
Xᵢ , for each i = 1 to k, may independently be selected from the group of -CH₂-CH₂-O-, -CH₂-CH₂-N(H)-, -CH₂-CH₂-CH₂-N(H)-, -CH₂-CH(NH₂)-, -CH₂-CH(NHCHO)-, -CH₂-CH(CH₃)-O-, -CH(CH₃)-CH₂-O-, -CH₂-C(CH₃)₂-O-, -C(CH₃)₂-CH₂-O-, -CH₂-CH₂-CH₂-O-, -CH₂-CH₂-CH₂- CH₂-O-, -CH₂-CHVin-O-, -CHVin-CH₂-O-, -CH₂-CHPh-O- and -CHPh-CH₂-O-, where Ph is phenyl and Vin is vinyl,
in the presence of at least one heterogeneous inorganic salt (S) are esterified with (meth)acrylic acid or transesterified with at least one (meth)acrylic ester (D).

2. The process according to claim 1, wherein the at least one inorganic salt (S) has a pK_{B} value of not more than 7.0 and not less than 1.0, and a solubility in the reaction medium at 25°C of not more than 1 g/l.

3. The process according to either of the preceding claims, wherein the inorganic salt has at least one anion selected from the group consisting of carbonate (CO₃²⁻), hydrogencarbonate (HCO₃⁻), phosphate (PO₄³⁻), hydrogenphosphate (HPO₄²⁻), dihydrogenphosphate (H₂PO₄⁻), sulfate (SO₄²⁻), hydrogensulfate (HSO₄⁻), sulfite (SO₃²⁻), hydrogensulfite (HSO₃⁻) and carboxylate (R⁶-COO⁻), where R⁶ is C₁ - C₁₈-alkyl, or C₂ - C₁₈-alkyl or C₆ - C₁₂-aryl optionally interrupted by one or more oxygen and/or sulfur atoms and/or one or more substituted or unsubstituted imino groups.

4. The process according to any of the preceding claims, wherein the inorganic salt has at least one cation selected from the group consisting of alkali metals, alkaline earth metals, ammonium, cerium, iron, manganese, chromium, molybdenum, cobalt, nickel or zinc.

5. The process according to any of the preceding claims, wherein the inorganic salt is selected from the group consisting of Li₃PO₄, K₃PO₄, Na₃PO₄ and K₂CO₃, and hydrates thereof.

6. The process according to any of the preceding claims, wherein R² is selected from the group consisting of 1,2-ethylene, 1,2-propylene, 1,1-dimethyl-1,2-ethylene, 1-hydroxymethyl-1,2-ethylene, 2-hydroxy-1,3-propylene, 1,3-propylene, 1,4-butylene, 1,6-hexylene, 2-methyl-1,3-propylene, 2-ethyl-1,3-propylene, 2,2-dimethyl-1,3-propylene and 2,2-dimethyl-1,4-butylene, 1,2-cyclopentylene, 1,3-cyclopentylene, 1,2-cyclohexylene, 1,3-cyclohexylene, ortho-phenylene, 3-oxa-1,5-pentylene, 3,6-dioxa-1,8-octylene and 3,6,8-trioxa-1,8,11-undecylene.

7. The process according to any of the preceding claims, wherein R¹ is selected from the group consisting of 1,2-ethylene, 1,2-propylene, 1,1-dimethyl-1,2-ethylene, 1-hydroxymethyl-1,2-ethylene, 2-hydroxy-1,3-propylene, 1,3-propylene, 1,4-butylene, 1,5-pentylene, 2-methyl-1,3-propylene, 2-ethyl-1,3-propylene, 2,2-dimethyl-1,3-propylene and 2,2-dimethyl-1,4-butylene.

8. The process according to any of the preceding claims, wherein (C) is selected from the group consisting of N-(2-hydroxyethyl)-pyrrolidone, N-(2-hydroxypropyl)-pyrrolidone, N-(2'-(2-hydroxyethoxy)ethyl)pyrrolidone, N-(2-hydroxyethyl)-caprolactam, N-(2-hydroxypropyl)caprolactam and N-(2'-(2-hydroxyethoxy)ethyl)-caprolactam.

## Revendications

1. Procédé de préparation de (méth)acrylates de lactames N-hydroxyalkylés, dans lequel des lactames cycliques, N-hydroxyalkylés (C), 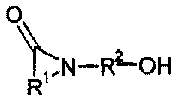 où
R¹ signifie C₁-C₅-alkylène ; ou C₂-C₂₀-alkylène interrompu par un ou plusieurs atomes d'oxygène et/ou de soufre et/ou par un ou plusieurs groupes imino substitués ou non substitués et/ou par un ou plusieurs groupes cycloalkyle, -(CO)-, -O(CO)O-, -(NH)(CO)O-, -O(CO) (NH)-, -O(CO)- ou -(CO)O-, les radicaux mentionnés pouvant à chaque fois être substitués par aryle, alkyle, aryloxy, alkyloxy, des hétéroatomes et/ou des hétérocycles, à condition que R¹ ne puisse pas présenter d'autre atome qu'un atome de carbone directement adjacent au groupe carbonyle du lactame,
R² signifie C₁-C₂₀-alkylène, C₅-C₁₂-cycloalkylène, C₆-C₁₂-arylène ; ou C₂-C₂₀-alkylène interrompu par un ou plusieurs atomes d'oxygène et/ou de soufre et/ou par un ou plusieurs groupes imino substitués ou non substitués et/ou par un ou plusieurs groupes cycloalkyle, -(CO)-, -O(CO)O-, -(NH)(CO)O-, -O(CO) (NH)-, -O(CO)- ou -(CO)O-, les radicaux mentionnés pouvant être à chaque fois substitués par aryle, alkyle, aryloxy, alkyloxy, des hétéroatomes et/ou des hétérocycles ou
R²-OH représente un groupe de formule -[Xᵢ]ₖ-H,
k vaut un nombre de 1 à 50 et
X₁ pour chaque i = 1 à k, peut être choisi indépendamment l'un de l'autre dans le groupe formé par -CH₂-CH₂-O-, -CH₂-CH₂-N (H)-, -CH₂-CH₂-CH₂-N(H)-, -CH₂-CH(NH₂)-, -CH₂-CH(NHCHO)-, -CH₂-CH(CH₃-0-, -CH(CH₃)-CH₂-O-, -CH₂-C(CH₃)₂-O-, -C(CH₃)₂-CH₂-O-, -CH₂-CH₂-CH₂-O-, -CH₂-CH₂-CH₂-CH₂-O-, -CH₂-CHVin-O-, -CHVin-CH₂-O-, -CH₂-CHPh-O- et -CHPh-CH₂-O-, où Ph représente phényle et Vin représente vinyle,
en présence d'au moins un sel (S) inorganique, hétérogène, sont estérifiés avec de l'acide (méth)acrylique ou transestérifiés avec au moins un ester de l'acide (méth)acrylique (D).

2. Procédé selon la revendications 1, **caractérisé en ce que** ledit au moins un sel inorganique (S) présente une valeur pKₐ qui n'est pas supérieure à 7,0 et pas inférieure à 1,0, ainsi qu'une solubilité dans le milieu réactionnel à 25°C qui n'est pas supérieure à 1 g/l.

3. Procédé selon l'une quelconque des revendications précédentes, **caractérisé en ce que** le sel inorganique présente au moins un anion choisi dans le groupe formé par carbonate (CO₃²⁻), hydrogénocarbonate (HCO₃⁻), phosphate (PO₄³⁻), hydrogénophosphate (HPO₄²⁻), dihydrogénophosphate (H₂PO₄⁻), sulfate (SO₄²⁻), hydrogénosulfate (HSO₄⁻), sulfite (SO₃²⁻), hydrogénosulfite (HSO₃⁻) et carboxylate (R⁶-CCO⁻), R⁶ signifiant C₁-C₁₀-alkyle ; ou C₂-C₁₈-alkyle ou C₆-C₁₂-aryle le cas échéant interrompu par un ou plusieurs atomes d'oxygène et/ou de soufre et/ou un ou plusieurs groupes imino substitués ou non substitués.

4. Procédé selon l'une quelconque des revendications précédentes, **caractérisé en ce que** le sel inorganique présente au moins un cation choisi dans le groupe constitué par les métaux alcalins, les métaux alcalino-terreux, l'ammonium, le cérium, le fer, le manganèse, le chrome, le molybdène, le cobalt, le nickel ou le zinc.

5. Procédé selon l'une quelconque des revendications précédentes, **caractérisé en ce que** le sel inorganique est choisi dans le groupe constitué par Li₃PO₄, K₃PO₄, Na₃PO₄ et K₂CO₃, ainsi que leurs hydrates.

6. Procédé selon l'une quelconque des revendications précédentes, **caractérisé en ce que** R² est choisi dans le groupe constitué par 1,2-éthylène, 1,2-propylène, 1,1-diméthyl-1,2-éthylène, 1-hydroxyméthyl-1,2-éthylène, 2-hydroxy-1,3-propylène, 1,3-propylène, 1,4-butylène, 1,6-hexylène, 2-méthyl-1,3-propylène, 2-éthyl-1,3-propylène, 2,2-diméthyl-1,3-propylène et 2,2-diméthyl-1,4-butylène, 1,2-cyclopentylène, 1,3-cyclopentylène, 1,2-cyclohexylène, 1,3-cyclohexylène, ortho-phénylène, 3-oxa-1,5-pentylène, 3,6-dioxa-1,8-octylène et 3,6,8-trioza-1,8,11-undécylène.

7. Procédé selon l'une quelconque des revendications précédentes, **caractérisé en ce que** R¹ est choisi dans le groupe constitué par 1,2-éthylène, 1,2-propylène, 1,1-diméthyl-1,2-éthylène, 1-hydroxyméthyl-1,2-éthylène, 2-hydroxy-1,3-propylène, 1,3-propylène, 1,4-butylène, 1,5-pentylène, 2-méthyl-1,3-propylène, 2-éthyl-1,3-propylène, 2,2-diméthyl-1,3-propylène et 2,2-diméthyl-1,4-butylène.

8. Procédé selon l'une quelconque des revendications précédentes, **caractérisé en ce que** (C) est choisi dans le groupe constitué par N-(2-hydroxyéthyl)-pyrrolidone, N-(2-hydroxypropyl)-pyrrolidone, N-(2'-(2-hydroxyéthoxy)-éthyl)-pyrrolidone, N-(2-hydroxyéthyl)-caprolactame, N-(2-hydroxypropyl)-caprolactame et N-(2'-(2-hydroxyéthoxy)-éthyl)-caprolactame.
